Europäisches Patentamt

European Patent Office

Office européen des brevets

(11) Veröffentlichungsnummer: **0 537 139 B1**

# (12) EUROPÄISCHE PATENTSCHRIFT

(45) Veröffentlichungstag der Patentschrift: **18.05.94**

(51) Int. Cl.5: **A61K 9/22**

(21) Anmeldenummer: **90909622.4**

(22) Anmeldetag: **02.07.90**

(86) Internationale Anmeldenummer:
**PCT/DE90/00498**

(87) Internationale Veröffentlichungsnummer:
**WO 92/00064 (09.01.92 92/02)**

(54) **VERFAHREN ZUR HERSTELLUNG GEFORMTER, VERPRESSTER DOSISEINHEITEN MIT RETARDIERTER FREISETZUNG UND ENTSPRECHENDE DOSISEINHEIT.**

(43) Veröffentlichungstag der Anmeldung:
**21.04.93 Patentblatt 93/16**

(45) Bekanntmachung des Hinweises auf die
Patenterteilung:
**18.05.94 Patentblatt 94/20**

(84) Benannte Vertragsstaaten:
**AT BE CH DE DK ES FR GB IT LI LU NL SE**

(56) Entgegenhaltungen:
**US-A- 3 629 393**
**US-A- 4 540 566**
**US-A- 4 608 248**

(73) Patentinhaber: **BOEHRINGER MANNHEIM GMBH**

**D-68298 Mannheim(DE)**

(72) Erfinder: **STEMMLE, Berthold**
**Velperstr. 14**
**D-3167 Burgdorf(DE)**
Erfinder: **BUDDE, Klaus**
**Plataner Str. 14**
**D-6143 Lorsch(DE)**
Erfinder: **WIRL, Alexander**
**Kurpfalzstr. 14**
**D-6711 Heuchelheim(DE)**
Erfinder: **DEMMER, Fritz**
**Kastanienweg 21**
**D-6945 Hirschberg-Leutershausen(DE)**

(74) Vertreter: **Pfeifer, Hans-Peter, Dr.rer.nat.**
**Patentanwalt**
**Nowackanlage 15**
**D-76137 Karlsruhe (DE)**

**Beschreibung**

Die Erfindung betrifft ein Verfahren zur Herstellung geformter, verpreßter Dosiseinheiten mit retardierter Freisetzung aus einem therapeutischen Wirkstoff, welcher preßdruckabhängig selbstretardierende Eigenschaften hat, sowie eine entsprechende Dosiseinheit.

Geformte, verpreßte Dosiseinheiten für medizinische und tiermedizinische Zwecke sind in zahlreichen Ausführungsformen bekannt. Der Begriff bezieht sich in erster Linie auf Tabletten, Filmtabletten oder Dragees. Die Erfindung ist aber auch auf Sonderformen therapeutischer Dosiseinheiten anwendbar. Im folgenden wird einfachheitshalber und ohne Beschränkung der Allgemeinheit beispielhaft auf Tabletten Bezug genommen.

Vielfach werden Tabletten mit retardierter Freisetzung entwickelt, um eine langsame und gleichmäßige Abgabe des therapeutischen Wirkstoffes über längere Zeit zu gewährleisten. Dadurch kann in vielen Fällen die Dauer der Wirkung eines Medikamentes verlängert oder zumindest die Einnahmevorschrift für den Patienten vereinfacht werden.

Besonders einfach läßt sich die Retardierung bei solchen Wirkstoffen realisieren, die preßdruckabhängig selbstretardierende Eigenschaften haben. Ein Beispiel eines solchen Wirkstoffes ist Bezafibrat, ein lipidsenkendes Medikament, das eine erhebliche Bedeutung bei der Therapie erhöhter Blutfettwerte erlangt hat. Dieser Wirkstoff bildet nadelförmige Kristallite, die sich ohne spezielle retardierende Hilfsstoffe zu Tabletten und anderen festen Dosiseinheiten mit retardierenden Eigenschaften verpressen lassen. Die Retardierungswirkung wird dabei über den Preßdruck eingestellt. Je stärker die Tablette gepreßt wird, desto langsamer ist die Freisetzung des Wirkstoffs. Diese Preßdruckabhängigkeit ist schon bei verhältnismäßig niedrigen Preßdrucken von etwa 10 kN/cm$^2$ festzustellen.

Nähere Einzelheiten über diesen Wirkstoff und geeignete Verfahren zu seiner Herstellung sind dem US-Patent 3 781 328 bzw. der DE-A-21 49 070 zu entnehmen.

Andere Wirkstoffe, die eine ausgeprägte Abhängigkeit der in-vitro-Freisetzungsrate von dem bei der Tablettierung angewandten Preßdruck aufweisen und deshalb preßdruckabhängig selbstretardierende Eigenschaften haben, sind die Verbindungen 4-[2-(-Chlorbenzolsulfonylamino)-ethyl]-phenylessigsäure und 4-[2-(Benzolsulfonylamino)ethyl]-phenoxyessigsäure, welche in den europäischen Anmeldungen EP-A-0 031 954 und EP-A-0 004 011 bzw. den US-Patenten 4 443 477 und 4 258 058 näher beschrieben werden.

Ein besonderer Vorteil der von dem Wirkstoff selbst ermöglichten Retardierung ist, daß nur geringe Hilfsstoffmengen erforderlich sind. Demzufolge können verhältnismäßig hohe Wirkstoffdosen in relativ kleinen Dosiseinheiten eingesetzt werden.

Trotz dieser positiven Eigenschaften können die bekannten Retard-Zubereitungen von Wirkstoffen der genannten Art nicht in jeder Hinsicht überzeugen. Insbesondere ergeben sich verhältnismäßig hohe Schwankungen der Retardeigenschaften. Dadurch wird eine sehr genaue Kontrolle der Parameter des Herstellungsprozesses erforderlich und das Risiko, daß Teile der Produktion wegen unzureichender pharmazeutischer Eigenschaften nicht verwendet werden können, steigt. Demgegenüber ist aus medizinischer Sicht eine möglichst gleichmäßige, weitgehend lineare und reproduzierbar gleichbleibende Freisetzung des Wirkstoffes wünschenswert.

Der Erfindung liegt daher die Aufgabe zugrunde, Retardzubereitungen der eingangs bezeichneten Art in den genannten Punkten zu verbessern. Zugleich soll dabei die insgesamt eingesetzte Hilfsstoffmenge möglichst wenig vergrößert werden, um auch bei hohen Wirkstoffmengen eine kompakte Dosiseinheit zu ermöglichen.

Die Aufgabe wird bei einem Verfahren der eingangs bezeichneten Art dadurch gelöst, daß in einer ersten Verfahrensstufe der Wirkstoff mit einem die Selbstretardierung hemmenden oder kompensierenden Hilfsstoffanteil derartig zu Partikeln verarbeitet wird, daß Preßlinge, die aus den Partikeln ohne weitere Zusätze hergestellt werden, in dem für die Pressung der Dosiseinheiten vorgesehenen Preßdruckbereich eine schnelle Freisetzung in Relation zu der gewünschten retardierten Freisetzung aufweisen und in einer zweiten Verfahrensstufe die Partikel mit einem Retardierungsmittel, das vorzugsweise ein Hydrogelbildner ist, welcher in einem prozentualen Anteil von 5 bis 40 Gewichtsprozent, bezogen auf die Wirkstoffmenge, enthalten ist, zu den Dosiseinheiten verpreßt werden. Weiter bevorzugt ist der Hydrogelbildner ein Cellulose-Derivat, insbesondere eine Methylhydroxypropylcellulose mit einem Molekulargewicht von weniger als 50.000 und einem Hydroxypropylgehalt von weniger als 9% oder eine Natriumcarboxymethylcellulose.

Eine erfindungsgemäße Dosiseinheit, welche miteinander verpreßte Partikel, die einen therapeutischen Wirkstoff enthalten, welcher Preßdruckabhängig retardierende Eigenschaften hat, und eine äußere Phase mit Tablettierungshilfsstoffen enthält, ist dadurch gekennzeichnet, daß die Partikel einen die Selbstretardierung des Wirkstoffes hemmenden oder kompensierenden Hilfsstoffanteil derartig enthalten, daß Preßlinge, die aus den Partikeln ohne weitere Zusätze hergestellt werden, in dem für die Pressung der Dosiseinheit vorgesehen Preßdruckbereich eine schnelle Freisetzung in Relation zu der gewünschten retardierten Freisetzung aufweisen, und die äußere Phase ein Retardierungsmittel enthält.

Die in der ersten Verfahrensstufe hergestellten Partikel sind vorzugsweise ein Granulat, welches bevorzugt durch Naßgranulation hergestellt wird. Alternativ können die Partikel aber auch durch andere bekannte Verfahren, insbesondere durch Trockengranulation (Brikettieren) oder als Pellets nach den bekannten Pelletisierungsverfahren hergestellt werden. Pellets, die sich in besonderem Maße für die Tablettierung eignen, sind Gegenstand der EP-A-0 218 928.

Die Größe der in der ersten Verfahrensstufe hergestellten Partikel kann in weiten Grenzen variieren. Verwendet man als Maßstab die "häufigst vorkommende Korngröße im RRSB-Netz" d', so liegt d' vorzugsweise zwischen ca. 0,2 und 0,5 mm. Der Streubereich der Teilchen kann zwischen etwa 0,01 mm und 2,0 mm liegen.

Der Fachmann kann auf Basis der hier vorliegenden Beschreibung geeignete Hilfsstoffe ohne weiteres auswählen, die die erwähnte Bedingung hinsichtlich der Relation der Freisetzung eines aus den Partikeln ohne weitere Zusätze hergestellten Preßlings und der gewünschten retardierten Freisetzung der Dosiseinheit (im folgenden kurz als "Freisetzungsbedingung" bezeichnet) erfüllt. Insbesondere kann der Wirkstoff mit großen Mengen an hydrophilen Hilfsstoffen, wie Zuckern, Zuckeralkoholen oder Polyethylenglykolen verarbeitet werden oder es können in der ersten Verfahrensstufe Hilfsstoffe mit großer Oberfläche, die eine Dochtwirkung in der Tablette ausüben, und Wasser bzw. Magen-Darmsäften das Eindringen in das Tablettengerüst erleichtern, verwendet werden.

Besonders bevorzugt sind jedoch die nachfolgend beschriebenen, spezifisch auf die Erfindung ausgerichteten Verfahrensweisen, die es erlauben, mit sehr geringen Hilfsstoffmengen zu arbeiten.

In der ersten Verfahrensstufe wird bevorzugt ein für Arzneimittel geeignetes und in der Arzneimittelindustrie gebräuchliches ("pharmazeutisches") Tablettenzerfallhilfsmittel, vorzugsweise in einer Konzentration zwischen 0,1 und 10 Gewichtsprozent, bevorzugt zwischen 0,25 und 5 Gewichtsprozent, bezogen auf die Wirkstoffmenge, eingesetzt. Der Begriff wird in dem Sinn gebraucht, wie er in dem Artikel P. H. List und U. A. Muazzam: "Quellung-die treibende Kraft beim Tablettenzerfall" Pharm. Ind., 41, Nr. 5, 459 ff (1979) diskutiert wird. Er bezeichnet also Substanzen, die den Zerfall von Aggregaten in diskrete Einzelteilchen, auch Primärteilchen genannt, beschleunigen, beispielsweise eine modifizierte Stärke, eine modifizierte Cellulose oder quervernetztes Polyvinylpyrrolidon. Im folgenden wird vereinfachend der gebräuchliche, aber weniger präzise Begriff "Sprengmittel" verwendet.

Eine andere bevorzugte Ausführungsform sieht vor, daß in der ersten Verfahrensstufe ein Netzmittel, vorzugsweise ein anionogenes Tensid, insbesondere Natriumdodecylsulfat, in einer Menge zwischen 0,1 und 10 Gewichtsprozent, bevorzugt zwischen 0,5 und 5 Gewichtsprozent Netzmittel, bezogen auf die Wirkstoffmenge, bevorzugt in Verbindung mit einem wasserlöslichen, pharmazeutischen Bindemittel, 2 dessen Konzentration bevorzugt zwischen 0,1 und 10 Gewichtsprozent, besonders bevorzugt zwischen 0,25 und 6 Gewichtsprozent an wasserlöslichem Bindemittel, bezogen auf die Wirkstoffmenge, beträgt, eingesetzt wird. Gerade durch diese Kombination wird eine insgesamt besonders gleichmäßige und auch bei verschiedenen Herstellungschargen der Tabletten reproduzierbar gleiche Freisetzung des Wirkstoffs erreicht.

Geeignete Hilfsstoffe, insbesondere Tablettenzerfallhilfsmittel, Netzmittel und wasserlösliche Bindemittel werden in pharmazeutischen Standardwerken, insbesondere "Pharmazeutische Technologie", herausgegeben von Heinz Sucker, Peter Fuchs und Peter Speiser, Georg Thieme-Verlag, Stuttgart (1978), angegeben.

Die Erfindung wird im folgenden anhand von experimentellen Daten, die in den beigefügten Figuren und Tabellen wiedergegeben sind, näher erläutert. Es zeigen:

Fig. 1    eine graphische Darstellung der in-vitro-Freisetzung bei Dosiseinheiten nach dem Stand der Technik,

Fig. 2    eine graphische Darstellung der in-vitro-Freisetzung bei erfindungsgemäßen Dosiseinheiten.

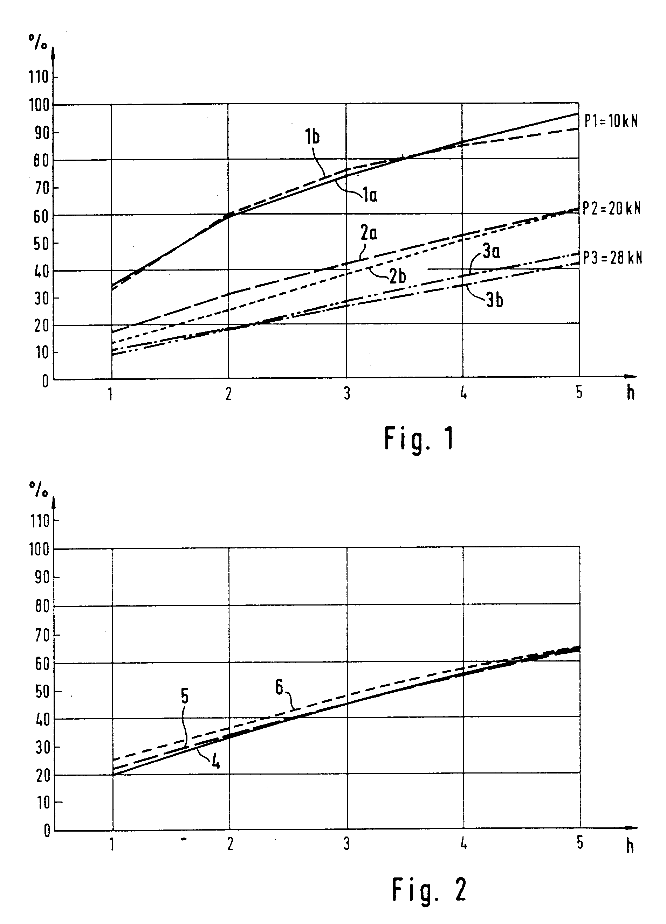

In Fig. 1 ist die in-vitro-Auflösungsrate (in Prozent) gegen die Zeit (in Stunden) für insgesamt sechs verschiedene Rezepturen einer Bezafibrat-Retard-Zubereitung nach dem Stand der Technik aufgetragen. Es handelt sich um Tabletten mit einem Durchmesser von 11 mm. Der Preßdruck betrug bei den Kurven 1a und 1b P1 = 10kN, bei den Kurven 2a und 2b P2 = 20kN und bei den Kurven 3a und 3b P3 = 28kN. Jeweils unterscheiden sich die beiden dargestellten Kurven hinsichtlich des Schüttvolumens des verwendeten Bezafibrats. Bei den mit a bezeichneten Kurven liegt das Schüttvolumen an der unteren Grenze des für Retardzubereitungen gebräuchlichen Bereiches (entsprechend ca. 0,4 m$^2$/g spezifische Oberfläche) und bei den mit b bezeichneten Kurven an der Obergrenze dieses Bereichs (entsprechend ca. 0,9m$^2$/g).

Aus Fig. 1 wird deutlich, daß die in-vitro-Auflösungsrate derartiger Retardtabletten in hohem Maße von dem Preßdruck abhängig ist. Dagegen ist der Einfluß des Schüttvolumens des Bezafibrats sehr gering.

Fig. 2 zeigt eine entsprechende Darstellung für eine erfindungsgemäße Rezeptur gemäß dem nachfolgenden Beispiel 7A. Die Tabletten hatten die gleiche Größe wie bei Fig. 1 und wurden bei den gleichen Preßdrucken verpreßt. Kurve 4 bezieht sich auf einen Preßdruck von 10 kN, Kurve 5 auf einen Preßdruck von 20 kN und Kurve 6 auf einen Preßdruck von 28 kN. Die in-vitro-Auflösungsrate ist nahezu unabhängig vom Preßdruck und verläuft praktisch linear. Der Verlauf ist auch bei verschiedenen Herstellungschargen ausgezeichnet reproduzierbar.

Zur Erläuterung der Freisetzungsbedingung, (daß die in der ersten Verfahrensstufe hergestellten Partikel einen solchen Anteil an einem die Selbstretardierung des Wirkstoffs hemmenden oder kompensierenden Hilfsstoff enthalten sollen, daß Preßlinge, die aus den Partikeln ohne weitere Zusätze hergestellt werden, in dem für die Pressung der Dosiseinheiten vorgesehenen Preßdruckbereich eine schnelle Freisetzung in Relation zu der gewünschten retardierten Freisetzung aufweisen), wird auf die folgenden Beispiele 1-4 und die zugehörige Tabelle 1 Bezug genommen.

Bei jedem der Beispiele 1-4 wurden die im folgenden angegebenen Bestandteile einer Rezeptur zur Erzeugung von zum nachfolgenden Tablettieren geeigneten Partikeln (Granulatrezeptur) trocken vermischt und zu einem Preßling mit einem Durchmesser von 11 mm trocken verpreßt. Bei den Versuchen erfolgte das Verpreßen mit einer üblichen schnell laufenden Tablettiermaschine. Dabei wurden dem Granulat übliche Tablettierhilfsstoffe (3 mg Siliciumdioxid, hochdispers und 10 mg Magnesium-Stearat) trocken beigemischt, um die Verarbeitungseigenschaften (Rieselfähigkeit, leichtes Lösen von der Form) zu verbessern. Die in-vitro-Auflösungsrate wird hierdurch praktisch nicht beeinflußt.

Als Maß für die Freisetzung des Wirkstoffes wurde die invitro-Freisetzungsrate unter folgenden Bedingungen bestimmt:
Prüfvolumen: 1.000 ml (Phosphatpuffer nach Küster Thiel)
pH : 6,8
Paddle-Rührer mit 90 min$^{-1}$
Temperatur : 37°C
Allgemein können für die Prüfung, ob eine bestimmte Rezeptur die Freisetzungsbedingung erfüllt, zweckmäßigerweise in-vitro-Verfahrensvorschriften verwendet werden. Dabei wird allgemein eine Verfahrensweise gewählt, die mit der in-vivo-Freisetzung korellierende Ergebnisse liefert. Selbstverständlich wird für die Bestimmung der Freisetzung der Preßlinge das gleiche in-vitro-Modell angewendet, wie bei der Bestimmung der Freisetzung der vollständigen (retardierten) Dosiseinheiten.

Beispiel 1:

| | |
|---|---|
| Bezafibrat | 400 mg |
| Mono- oder Oligosaccharid (hier Lactose D80) | 51 mg |
| Poly(vinylpyrrolidon) 25.000 | 15 mg |

Diese Rezeptur enthält das als Füllstoff übliche Oligosaccharid. Es dient in bekannter Weise als Füllstoff bei der Granulatbildung und erleichtert die Kompression des Granulats zu Tabletten. Das Polyvinylpyrrolidon ist ein wasserlösliches Polymer, welches als Bindemittel in Granulatrezepturen gebräuchlich ist.

Die Rezeptur enthält also keinerlei Hilfsstoffe, die spezifisch darauf ausgerichtet sind, die Selbstretardierung des Wirkstoffs Bezafibrat zu hemmen oder zu kompensieren. Tabelle 1 zeigt, daß in der Tat die in-vitro-Auflösungsrate sehr langsam ist und eine erhebliche Druckabhängigkeit zeigt. Nach 5 Std. wird eine 23%ige bzw. 33%ige Auflösung festgestellt, also erheblich weniger als bei dem (im Beispiel gem. Fig. 2) gewünschten Verhalten der Retardzubereitung.

Beispiel 2:

| | |
|---|---|
| Bezafibrat | 400 mg |
| Mono- oder Oligosaccharid (hier Lactose D80) | 51 mg |
| Natriumdodecylsulfat (Netzmittel) | 10 mg |

Das Netzmittel Natriumdodecylsulfat führt bei niedrigen Preßdrucken zu einer deutlichen Erhöhung der in-vitro-Auflösungsgeschwindigkeit. Diese ist jedoch weiterhin stark preßdruckabhängig. Außerdem lassen die mechanischen Eigenschaften der Preßlinge zu wünschen übrig.

Beispiele 3A und 3B:

|  | 3A | 3B |
|---|---|---|
| Bezafibrat | 400 mg | 400 mg |
| Mono- oder Oligosaccharid (hier Mannit) | 53 mg | 53 mg |
| Poly(vinylpyrrolidon) 25.000 | 15 mg | 15 mg |
| Natriumcarboxymethylstärke | 4 mg | 8 mg |

Diese Rezeptur enthält in zwei verschiedenen Konzentrationen das Sprengmittel Natriumcarboxymethylstärke. Tabelle 1 zeigt, daß hierdurch eine schnelle und preßkraftunabhängige in-vitro-Auflösung der Preßlinge erreicht wird. Bei diesen Beispielen ist die für die Erfindung erforderliche Bedingung ohne weiteres erfüllt. Man erkennt, daß bereits eine sehr kleine Konzentration von Sprengmittel (1% der Wirkstoffmenge) ausreicht. Das Tablettenvolumen wird hierdurch praktisch nicht vergrößert.

Beispiel 4:

|  |  |  |
|---|---|---|
| Bezafibrat | 400 mg | 400 mg |
| Mono- oder Oligosaccharid (hier Lactose D80) | 51 mg | 51 mg |
| Poly(vinylpyrrolidon) 25.000 | 15 mg | 15 mg |
| Natriumdodecylsulfat | 10 mg |  |
| Macrogol-Stearat 2500 |  | 10 mg |

In diesen Rezepturen wird gleichzeitig ein wasserlösliches polymeres Bindemittel (Polyvinylpyrrolidon) und ein Netzmittel eingesetzt, wobei in Beispiel 4B das anionogene Natriumdodecylsulfat ersetzt wird durch ein nichtionogenes Netzmittel (Macrogol-Stearat 2.500). Tabelle 1 zeigt, daß die Freisetzungsbedingung für die erstgenannte Kombination (Beispiel 4A) für sämtliche Preßdrucke erfüllt wird, während beim Beispiel 4B eine knapp ausreichende Auflösung der Preßlinge nur für verhältnismäßig niedrige Preßdrucke gewährleistet ist.

Aus den Beispielen 1-4 läßt sich insgesamt folgendes ableiten:

- Es ist möglich, mit Hilfe verhältnismäßig geringer Mengen von die Selbstretardierung hemmenden oder kompensierenden Hilfsstoffen aus Bezafibrat Preßlinge herzustellen, die bei üblichen Tablettierungspreßdrucken eine schnelle Freisetzung in Relation zu der gewünschten Retardierung der Dosiseinheit aufweisen.
- Dies läßt sich besonders wirksam durch Verwendung eines Sprengmittels erreichen. Sehr gute Ergebnisse erhält man auch durch eine Kombination eines wasserlöslichen Bindemittels mit einem, vorzugsweise anionogenen, Tensid.

Die Beispiele 5-8 dienen dazu, die Wirksamkeit der Erfindung in Verbindung mit Methylhydroxipropylcellulose (MHPC) als Retardierungsmittel zu belegen.

Bei den Beispielen 5-7A werden jeweils 400 mg Bezafibrat mit wasserlöslichem Bindemittel und entweder Sprengmittel oder Tensid entsprechend den obigen Beispielen 3B,4A und 4B naß granuliert. Das Granulat wird mit den als äußere Phase angegebenen Bestandteilen zu Tabletten mit einem Durchmesser von 11 mm verpreßt. Die in-vitro-Freisetzungsrate wird wie zuvor beobachtet.

Beispiele 5,6 und 7A:

| Granulat: | 5 | 6 | 7A |
|---|---|---|---|
| Bezafibrat | 400 mg | 400 mg | 400 mg |
| Lactose D80 | | 53 mg | 51 mg |
| Mannit | 53 mg | | |
| Poly(vinylpyrrolidon) 25.000 | 15 mg | 15 mg | 15 mg |
| Natriumcarboxymethylstärke | 8 mg | | |
| Natriumdodecylsulfat | | | 10 mg |
| Macrogol-Stearat 2.500 | | 10 mg | |
| äußere Phase: | | | |
| MHPC K 100 LV | 51 mg | 49 mg | 51 mg |
| Siliciumdioxid,hochdispers | 3 mg | 3 mg | 3 mg |
| Magnesium-Stearat | 10 mg | 10 mg | 10 mg |

Die Ergebnisse sind in Tabelle 2 wiedergegeben. Es zeigt sich, daß in allen drei Fällen eine weitgehend preßkraftunabhängige Freisetzung des Wirkstoffs erzielt wird. Das Freisetzungsverhalten ist bei Beispiel 5 und bei Beispiel 6 ähnlich. Hinsichtlich Bruchfestigkeit und Abrieb ist jedoch die erstgenannte Zusammensetzung überlegen. Überraschenderweise zeigt sich, daß die nach den Beispielen 1-4 zwar geeignet, aber weniger bevorzugt erscheinende Granulatrezeptur mit einem anionogenem Tensid und einem wasserlöslichen Polymer eine besonders gleichmäßige, lineare und druckunabhängige Freisetzung zeigt. Zugleich werden voll befriedigende mechanische Eigenschaften erzielt. Von besonderem Vorteil ist die geringe Schwankung der Freisetzung, die sich in den in Klammern hinter der in-vitro-Auflösungsrate angegebenen Werten des Variationskoeffizienten (VK) ausdrückt. Das Auflösungsverhalten dieser Rezeptur ist in Fig. 2 dargestellt.

An diesem Beispiel wird auch deutlich, daß die Freisetzungsbedingung nicht dahingehend zu verstehen ist, daß über den gesamten Zeitraum der retardierten Auflösung die momentane Auflösungsgeschwindigkeit des Preßlings höher sein muß als die der fertigen Tablette. Im vorliegenden Fall ist sie innerhalb der ersten Stunde für die höheren Preßdrucke praktisch gleich, wie sich aus dem Vergleich von Tabelle 1, Beispiel 4A mit Tabelle 2, Beispiel 7A ergibt.

Erforderlich ist jedoch, daß sich die Auflösung innerhalb eines für Retard-Zubereitungen typischen Zeitraums signifikant unterscheidet. Quantitativ kann man sagen, daß nach drei Stunden die Auflösung des Preßlings mindestens 1,5 mal so weit fortgeschritten sein sollte, wie die Auflösung der fertigen Tablette.

Die Beispiele 7B - 7E zeigen die retardierende Wirkung veschiedener Konzentrationen von MHPC bei einer Rezeptur, die im übrigen dem besonders bevorzugten Beispiel 7A entspricht. Dabei wurden folgende Konzentrationen von MHPC verwendet.

| 7B | 7C | 7D | 7E |
|---|---|---|---|
| 31 mg | 41 mg | 71 mg | 101 mg |

Aus den Werten der Tabelle 2 erkennt man, daß bei sehr geringen Anteilen an MHPC (bezogen auf die Wirkstoffmenge weniger als etwa 10 %) die Retardierung preßkraftabhängig ist. Es ist aber nicht die aufgrund des Verhaltens des reinen Wirkstoffes zu erwartende Reduzierung der in-vitro-Auflösungsraten bei steigendem Preßdruck festzustellen. Im Gegenteil lösen sich die Tabletten bei höheren Preßdrucken schneller auf.

Mit MHPC-Konzentrationen oberhalb des genannten Grenzwertes läßt sich die Retardierung in weiten Bereichen wunschgemäß einstellen.

Um den Einfluß des Tensids zu differenzieren, wurde in Beispiel 8 eine Rezeptur ohne Tensid, jedoch mit wasserlöslichem Polymer erprobt:

Beispiel 8:

| Granulat: | |
|---|---|
| Bezafibrat | 400 mg |
| Mono- oder Oligosaccharid (hier Lactose D80) | 51 mg |
| Poly(vinylpyrrolidon) 25.000 | 15 mg |
| äußere Phase: | |
| MHPC K 100 LV | 51 mg |
| Siliciumdioxid, hochdispers | 3 mg |
| Magnesium-Stearat | 10 mg |

Tabelle 2 zeigt, daß die Preßkraftabhängigkeit der Retardierung in diesem Falle zwar vermindert, aber dennoch in störendem Maße vorhanden ist. Vor allem aber sind sehr hohe Variationskoeffizienten festzustellen. Die Eigenschaften der Rezeptur sind also nicht gut reproduzierbar.

Insgesamt zeigt sich, daß eine Rezeptur mit wasserlöslichem Bindemittel (insbesondere Polyvinylpyrrolidon), anionogenem Tensid (insbesondere Natriumdodecylsulfat) in der ersten Verfahrensstufe (Partikel) und Gelmatrixbildner (insbesondere MHPC) als Retardierungsmittel in der zweiten Verfahrensstufe (äußere Phase) besonders vorteilhafte Wirkungen hat. Die Qualität der Retardierung ist besser, als dies aufgrund des Löslichkeitsverhaltens der Preßlinge zu erwarten ist. Man kann deswegen von einer synergistischen Wirkung sprechen, die vermutlich auf eine Wechselwirkung zwischen dem anionogenem Netzmittel und dem MHPC zurückzuführen ist.

Die Erfindung kann in ihrer allgemeinen Form jedoch vorteilhaft auch mit anderen Kombinationen von Hilfsstoffen, insbesondere mit anderen bekannten Retardierungsmitteln verwendet werden. In einer Vielzahl von Versuchen hat sich insbesondere Natrium-Alginat als gut brauchbar erwiesen. Unter den Cellulose-Derivaten ist Natrium-Carboxymethylcellulose geeignet.

Als wasserlösliches Bindemittel eignen sich statt des Poly(vinylpyrrolidons) auch Cellulosederivate (z.B. niedrigviskose Methylhydroxypropylcellulosen, niedrigsubstituierte Hydroxypropylcellulosen, Zuckerester oder feste Polyethylenglykole).

Hinsichtlich des Tensids sind die Alternativen beschränkt. Unter den ionischen Tensiden ist nur das Natriumdodecylsulfat als pharmazeutischer Hilfsstoff zugelassen. Unter den nichtionischen Tensiden können beispielsweise auch Macrogol-Stearate (z.B. Macrogol(50)-Stearat), Polysorbate (z.B. Polysorbat 80) oder Polyoxyethylenpolyoxypropylen-Copolymere (z.B. Pluronic F 68) verwendet werden.

Wegen der Vielzahl der zur Verfügung stehenden pharmazeutischen Hilfsstoffe ist es nicht möglich, eine Regel zur unmittelbaren Auswahl der in der ersten Verfahrensstufe (Partikel) zu verwendenden Hilfsstoffe und der in der zweiten Verfahrensstufe (äupere Phase) zu verwendenden Retardierungsmittel anzugeben. Aufgrund der in dieser Erfindungsbeschreibung gegebenen Hinweise kann der Fachmann jedoch planmäßig unter den zur Verfügung stehenden Hilfsstoffen seine Auswahl treffen und die Eignung durch einfache Versuche erproben.

Unter den verschiedenen MHPC-Typen, die am Markt verfügbar sind, hat sich ein Produkt mit folgenden Werten besonders bewährt:
Gehalt an Hydroxypropyl: weniger als 9 %
mittleres Molekulargewicht ca. 26.000
mittlere Viskosität einer 2%igen Lösung bei 20 °C: 0,1 Pa sec.

Tabelle 1:

| Bsp Nr. | Preß-kraft [KN] | Bruchfest-igkeit [N] | Abrieb [%] | In-vitro-Auflösungsrate (VK) [%] 1[h] | 2[h] | 3[h] | [%] 4[h] | 5[h] |
|---|---|---|---|---|---|---|---|---|
| 01 | 10 | 129 | 0,55 | 6 ( 5,8) | 8 ( 9,4) | 16 ( 6) | 20 ( 7,5) | 33 ( 9,5) |
|  | 20 | 144 | 0,52 | – | – | – | – | – |
|  | 28 | 153 | 0,54 | 4 (13,9) | 11 ( 4,9) | 12 ( 5,7) | 13 (11,5) | 23 ( 8,8) |
| 02 | 10 | 54 | deckeln | 35 (65,5) | 61,0(38,9) | 71,0(29,3) | 76,0(25,7) | 79,0(23,5) |
|  | 20 | 57 | deckeln | 12 ( 6,4) | 19,0( 6,1) | 24,0( 7,2) | 28,0( 8,4) | 31,0( 9,1) |
|  | 28 | 58 | deckeln | 12 ( 2,3) | 19,0( 2,6) | 23,0( 2,9) | 26,0( 3,6) | 29,6( 3,9) |
| 03A | 10 | 109 | 0,45 | 97 ( 0,9) | 98,0( 0,7) | 98,0( 0,9) | 99,0( 0,7) | 100,0( 0,7) |
|  | 20 | 167 | 0,39 | 94 ( 1,6) | 99,0( 0,8) | 99,0( 0,7) | 100,0( 0,8) | 100,0( 0,9) |
|  | 28 | – | – | – | – | – | – | – |
| 03B | 10 | 106 | – | 98 ( 1,0) | 98 ( 1,2) | 98 ( 1,2) | 98 ( 1,2) | 99 ( 0,9) |
|  | 20 | 164 | 0,3 | 97 ( 1,0) | 97 ( 1,0) | 98 ( 1,0) | 98 ( 1,1) | 98 ( 1,0) |
|  | 28 | 165 | – | – | – | – | – | – |
| 04A | 10 | 136 | 0,55 | 70 ( 4,7) | 99 ( 1,0) | 100 ( 0,7) |  |  |
|  | 20 | 188 | 0,62 | 21 ( 4,0) | 60 (14,2) | 100 ( 3,9) |  |  |
|  | 28 | 197 | 0,51 | 20 ( 3,8) | 61 ( 7,2) | 96 ( 1,0) |  |  |
| 04B | 10 | 80 | 0,81 | 20 (14,2) | 49,0(21,1) | 76,0(20,5) | 91,0( 8,9) | 98,0( 1,9) |
|  | 20 | 94 | 0,69 | 16 ( 3,4) | 29,0( 4,4) | 41,0( 6,2) | 53,0( 8,7) | 64,0(10,0) |
|  | 28 | 100 | 0,55 | 16 ( 3,8) | 29,0( 2,3) | 40,0( 1,9) | 51,0( 2,0) | 62,0( 2,3) |

EP 0 537 139 B1

Tabelle 2:

| Bsp Nr. | Preß-kraft [KN] | Bruchfest-igkeit [N] | Abrieb [%] | In-vitro-Auflösungsrate (VK) [%] | | | | |
|---|---|---|---|---|---|---|---|---|
| | | | | 1[h] | 2[h] | 3[h] | 4[h] | 5[h] |
| 05 | 10 | 163 | 0,30 | 38,0(10,1) | 54,0( 8,2) | 71 ( 8,7) | 84,0( 9,3) | 96,0( 6,9) |
| | 20 | 231 | 0,30 | 48,0(16,7) | 65,0(13,1) | 75 (11,6) | 85,0(10,5) | 97,0( 5,7) |
| | 28 | 262 | 0,30 | 44,0(18,3) | 59,0(14,2) | 71 (13,4) | 81,0(10,8) | 90,0( 8,5) |
| 06 | 10 | 52 | 0,6 | 46,0( 9,4) | 63,0( 9,2) | 78 ( 9,7) | 91,0( 8,7) | 100,0( 4,9) |
| | 20 | 95 | 0,4 | 54,0( 9,8) | 67,0( 8,7) | 78 ( 7,2) | 89,0( 8,8) | 97,0( 6,2) |
| | 28 | 111 | 0,4 | 59,0(25,9) | 71,0(24,6) | 80 (17,7) | 87,0(12,7) | 95,0( 9,5) |
| 07A | 10 | 98 | 0,7 | 20,0( 6,6) | 33,0( 5,1) | 45,0( 5,1) | 55,0( 5,5) | 65,0( 5,6) |
| | 20 | 160 | 0,3 | 22,0( 5,9) | 34,0( 3,6) | 45,0( 4,4) | 55,0( 3,6) | 64,0( 4,3) |
| | 28 | 180 | 0,5 | 25,0( 6,9) | 36,0( 6,5) | 48,0( 6,0) | 57,0( 9,8) | 65,0( 6,5) |
| 07B | 10 | 146 | 0,5 | 35,0(17,1) | 54,0(15,6) | 72,0(17,6) | 89,0(11,7) | 96,0( 7,4) |
| | 20 | 171 | 0,4 | 61,0(15,7) | 88,0(14,8) | 94,0( 9,5) | 98,0( 4,9) | 100,0( 2,2) |
| | 28 | 200 | 0,4 | 66,0( 4,3) | 91,0( 6,1) | 99,0( 3,6) | 101,0( 1,1) | 102,0( 0,8) |
| 07C | 10 | 121 | 0,3 | 24,0( 7,0) | 38,0( 5,2) | 49,0( 4,5) | 59,0( 4,4) | 69,0( 4,5) |
| | 20 | 168 | – | 45,0(14,7) | 56,0(12,0) | 66,0( 9,2) | 76,0(10,7) | 85,0(10,5) |
| | 28 | 164 | 0,2 | 49,0(13,2) | 60,0(11,8) | 68,0(10,8) | 77,0(13,0) | 86,0(11,9) |
| 07D | 10 | 136 | 0,2 | 14,0(29,8) | 29,0(20,2) | 43,0(18,0) | 53,0(18,1) | 63,0(17,7) |
| | 20 | 195 | 0,3 | 15,0( 8,2) | 30,0( 7,1) | 45,0( 7,2) | 56,0( 6,7) | 66,0( 6,1) |
| | 28 | 208 | 0,2 | 16,0(10,4) | 31,0( 8,4) | 45,0(10,1) | 56,0( 9,2) | 67,0( 8,5) |
| 07E | 10 | 133 | 0,2 | 10,0(21,1) | 22,0(18,1) | 34,0(16,0) | 44,0(15,2) | 53,0(14,5) |
| | 20 | 206 | 0,2 | 10,0(23,5) | 22,0(21,8) | 34,0(20,2) | 44,0(18,1) | 53,0(16,9) |
| | 28 | 229 | 0,2 | 10,0( 7,7) | 22,0( 6,2) | 34,0( 6,0) | 45,0( 5,7) | 55,0( 5,4) |
| 08 | 10 | 114 | 0,1 | 33,0(23,3) | 50,0(20,1) | 63,0(18,7) | 75,0(17,4) | 85,0(15,8) |
| | 20 | 170 | 0,1 | 31,0(21,3) | 55,0(19,2) | 69,0(18,2) | 81,0(17,7) | 89,0(17,0) |
| | 28 | 180 | 0,2 | 34,0(19,2) | 69,0(13,5) | 85,0( 8,9) | 94,0( 5,7) | 100,0( 3,4) |

**Patentansprüche**

1. Verfahren zur Herstellung geformter, verpreßter Dosiseinheiten mit retardierter Freisetzung aus einem therapeutischen Wirkstoff, welcher preßdruckabhängig selbstretardierende Eigenschaften hat,

**dadurch gekennzeichnet**, daß
in einer ersten Verfahrensstufe der Wirkstoff mit einem die Selbstretardierung hemmenden oder kompensierenden Hilfsstoffanteil derartig zu Partikeln verarbeitet wird, daß Preßlinge, die aus den Partikeln ohne weitere Zusätze hergestellt wurden, in dem für die Pressung der Dosiseinheiten vorgesehenen Preßdruckbereich eine schnelle Freisetzung in Relation zu der gewünschten retardierten Freisetzung aufweisen und
in einer zweiten Verfahrensstufe die Partikel mit einem Retardierungsmittel zu den Dosiseinheiten verpreßt werden.

2. Verfahren nach Anspruch 1, **dadurch gekennzeichnet**, daß der Hilfsstoffanteil in der ersten Verfahrensstufe ein pharmazeutisches Tablettenzerfallhilfsmittel enthält.

3. Verfahren nach Anspruch 2, **dadurch gekennzeichnet**, daß das Tablettenzerfallhilfsmittel in einer Konzentration zwischen 0,1 und 10 Gewichtsprozent bevorzugt zwischen 0,25 und 5 Gewichtsprozent, bezogen auf die Wirkstoffmenge, eingesetzt wird.

4. Verfahren nach Anspruch 1, **dadurch gekennzeichnet**, daß der Hilfsstoffanteil in der ersten Verfahrensstufe ein Netzmittel enthält.

5. Verfahren nach Anspruch 4, **dadurch gekennzeichnet**, daß das Netzmittel ein anionogenes Tensid, insbesondere Natriumdodecylsulfat ist.

6. Verfahren nach Anspruch 4 oder 5, **dadurch gekennzeichnet**, daß zwischen 0,1 und 10 Gewichtsprozent, bevorzugt zwischen 0,5 und 5 Gewichtsprozent Netzmittel, bezogen auf die Wirkstoffmenge, eingesetzt werden.

7. Verfahren nach Anspruch 4,5 oder 6 **dadurch gekennzeichnet**, daß der Hilfsstoffanteil in der ersten Verfahrensstufe ein pharmazeutisches Bindemittel enthält.

8. Verfahren nach Anspruch 6, **dadurch gekennzeichnet**, daß zwischen 0,1 und 10 Gewichtsprozent, bevorzugt zwischen 0,25 und 6 Gewichtsprozent an wasserlöslichem Bindemittel, bezogen auf die Wirkstoffmenge, eingesetzt werden.

9. Verfahren nach Anspruch 1, **dadurch gekennzeichnet**, daß das Retardierungsmittel ein Hydrogelbildner ist, welcher in einem prozentualen Anteil von 5 bis 40 Gewichtsprozent, bezogen auf die Wirkstoffmenge, eingesetzt wird.

10. Verfahren nach Anspruch 9, **dadurch gekennzeichnet**, daß der Hydrogelbildner ein Cellulose-Derivat, insbesondere eine Methylhydroxypropylcellulose mit einem Molekulargewicht von weniger als 50.000 und einem Hydroxypropylgehalt von weniger als 9% oder eine Natriumcarboxymethylcellulose ist.

11. Verfahren nach Anspruch 1, **dadurch gekennzeichnet**, daß das Retardierungsmittel ein Alginat, insbesondere ein Natriumalginat ist.

12. Verfahren nach einem der vorhergehenden Ansprüche, **dadurch gekennzeichnet**, daß der therapeutische Wirkstoff Bezafibrat ist.

13. Geformte, verpreßte Dosiseinheit mit retardierter Freisetzung, enthaltend
miteinander verpreßte Partikel, die einen therapeutischen Wirkstoff enthalten, welcher preßdruckabhängig retardierende Eigenschaften hat, und
eine äußere Phase mit Tablettierungshilfsstoffen,
**dadurch gekennzeichnet**, daß
die Partikel einen die Selbstretardierung des Wirkstoffes hemmenden oder kompensierenden Hilfsstoffanteil derartig enthalten, daß Preßlinge, die aus den Partikeln ohne weitere Zusätze hergestellt werden, in dem für die Pressung der Dosiseinheit vorgesehenen Preßdruckbereich eine schnelle Freisetzung in Relation zu der gewünschten retardierten Freisetzung aufweisen, und
die äußere Phase ein Retardierungsmittel enthält.

**14.** Dosiseinheit nach Anspruch 12, **dadurch gekennzeichnet**, daß der therapeutische Wirkstoff Bezafibrat ist.

**Claims**

**1.** Process for preparing shaped, press-moulded unit doses that are subject to retarded release from a therapeutic active substance having self-retarding properties that vary with compacting pressure, characterised in that
in a first processing stage the active substance is worked into particles with an auxiliary substance that inhibits or compensates the self-retardation in such a way that pressed articles produced from the particles without any further additives in the compacting-pressure range specified for compression moulding of the unit doses exhibit a rapid release in relation to the desired retarded release
and
in a second processing stage the particles are press-moulded with a retarding agent to form the unit doses.

**2.** Process according to Claim 1, characterised in that the proportion of auxiliary substance in the first processing stage contains a pharmaceutical auxiliary substance that promotes the disintegration of tablets.

**3.** Process according to Claim 2, characterised in that the auxiliary substance that promotes the disintegration of tablets is used in a concentration between 0.1 and 10 per cent by weight, preferably between 0.25 and 5 per cent by weight, relative to the quantity of active substance.

**4.** Process according to Claim 1, characterised in that the proportion of auxiliary substance in the first processing stage contains a wetting agent.

**5.** Process according to Claim 4, characterised in that the wetting agent is an anionic surfactant, in particular sodium dodecyl sulphate.

**6.** Process according to Claim 4 or 5, characterised in that use is made of between 0.1 and 10 per cent by weight, preferably between 0.5 and 5 per cent by weight of wetting agent, relative to the quantity of active substance.

**7.** Process according to Claim 4, 5 or 6, characterised in that the proportion of auxiliary substance in the first processing stage contains a pharmaceutical binding agent.

**8.** Process according to Claim 6, characterised in that use is made of between 0.1 and 10 per cent by weight, preferably between 0.25 and 6 per cent by weight of water-soluble binding agent, relative to the quantity of active substance.

**9.** Process according to Claim 1, characterised in that the retarding agent is a hydrogel-forming agent which is used in a percentage proportion of 5 to 40 per cent by weight, relative to the quantity of active substance.

**10.** Process according to Claim 9, characterised in that the hydrogel-forming agent is a cellulose derivative, in particular a methylhydroxypropyl cellulose with a molecular weight of less than 50,000 and a hydroxypropyl content of less than 9%, or a sodium carboxymethyl cellulose.

**11.** Process according to Claim 1, characterised in that the retarding agent is an alginate, in particular a sodium alginate.

**12.** Process according to one of the preceding claims, characterised in that the therapeutic active substance is bezafibrate.

**13.** Shaped, press-moulded unit dose that is subject to retarded release, containing
particles which are jointly press-moulded and which contain a therapeutic active substance that has retarding properties varying with compacting pressure, and an outer phase containing auxiliary

11

substances used in the production of tablets,
characterised in that
the particles contain a proportion of auxiliary substance that inhibits or compensates the self-retardation of the active substance in such a way that pressed articles produced from the particles without any further additives in the compacting-pressure range specified for the compression moulding exhibit a rapid release in relation to the desired retarded release, and
the outer phase contains a retarding agent.

14. Unit dose according to Claim 12, characterised in that the therapeutic active substance is bezafibrate.

**Revendications**

1. Procédé de préparation de doses unitaires comprimées, moulées, a effet retard, constituées d'une substance thérapeutiquement active, qui présente des propriétés d'autoretardement dépendantes de la pression appliquée, caractérisé en ce que
dans une première étape du procédé, on transforme la substance active en particules, avec un adjuvant inhibant ou compensant l'auto-retardement, de telle façon que les ébauches, préparées à partir des particules sans autre adjonction, présentent, dans la gamme des pressions appliquées prévues pour la compression des doses unitaires, une libération rapide en relation avec la libération retardée souhaitée, et
dans une seconde étape du procédé, on compresse les particules en doses unitaires, avec un agent de retardement.

2. Procédé selon la revendication 1, caractérisé en ce que la partie adjuvant contient, dans la première étape du procédé, un adjuvant de désintégration pour comprimés pharmaceutiques.

3. Procédé selon la revendication 2, caractérisé en ce que l'adjuvant de désintégration pour comprimés est mis en oeuvre en une concentration comprise entre 0,1 et 10 % en poids, de préférence entre 0,25 et 5 % en poids, par rapport à la quantité de substance active.

4. Procédé selon la revendication 1, caractérisé en ce que la partie adjuvant contient, dans la première étape du procédé, un agent mouillant.

5. Procédé selon la revendication 4, caractérisé en ce que l'agent mouillant est un tensioactif anionogène, en particulier le dodécylsulfate de sodium.

6. Procédé selon la revendication 4 ou 5, caractérisé en ce que l'on met en oeuvre une proportion de 0,1 à 10 % en poids, de préférence de 0,5 à 5 % en poids, d'agent mouillant, par rapport à la quantité de substance active.

7. Procédé selon l'une quelconque des revendications 4, 5 ou 6, caractérisé en ce que la partie adjuvant de la première étape du procédé contient un liant pharmaceutique.

8. Procédé selon la revendication 6, caractérisé en ce que l'on met en oeuvre une proportion de 0,1 à 10 % en poids, de préférence 0,25 à 6 % en poids, de liant hydrosoluble, par rapport à la quantité de substance active.

9. Procédé selon la revendication 1, caractérisé en ce que l'agent de retardement est un formateur d'hydrogel, qui est utilisé dans une proportion de 5 à 40 % en poids par rapport à la substance active.

10. Procédé selon la revendication 9, caractérisé en ce que le formateur d'hydrogel est un dérivé cellulosique, en particulier une méthylhydroxypropylcellulose ayant une masse moléculaire inférieure à 50 000 et une teneur en hydroxypropyle inférieure à 9 %, ou une carboxyméthylcellulose sodique.

11. Procédé selon la revendication 1, caractérisé en ce que l'agent de retardement est un alginate, en particulier l'alginate de sodium.

**12.** Procédé selon l'une quelconque des revendications précédentes, caractérisé en ce que la substance thérapeutiquement active est le bézafibrate.

**13.** Dose unitaire comprimée, moulée, ayant un effet retard, contenant

des particules compressées entre elles, contenant une substance thérapeutiquement active, qui présente des propriétés de retardement dépendant de la pression appliquée, et
une phase extérieure comportant des adjuvants utilisés pour la préparation de comprimés, caractérisée en ce que
les particules contiennent un adjuvant inhibant ou compensant l'auto-retardement de la substance active de façon que les ébauches, qui ont été préparées à partir des particules sans autre adjonction, présentent une libération rapide dans la gamme des pressions appliquées prévues pour la compression des doses unitaires, en relation avec la libération retardée souhaitée, et
la phase extérieure contient un agent de retardement.

**14.** Dose unitaire selon la revendication 12, caractérisée en ce que la substance thérapeutiquement active est le bézafibrate.

Fig. 1

Fig. 2

14